# EUROPEAN PATENT APPLICATION

(11) **EP 3 970 783 A1**
(43) Date of publication of application: **23.03.2022**
(21) Application number: 21197482.9
(22) Date of filing: 17.09.2021
(51) Int. Cl.: A61M 39/02, A61B 17/00, A61M 25/00

(54) **CONVERTIBLE INTRODUCER SHEATH**

(30) Priority: 19.09.2020 US 202063080716 P; 15.09.2021 US 202117476450
(71) Applicant: Tau-PNU Medical Co., Ltd., Busan 46241 (KR)
(72) Inventor: KIM, June Hong, Busan (KR); CURLEY, Jimmy, Yangsan-si (KR); PARK, Kyone, Yangsan (KR)
(74) Representative: dompatent von Kreisler Selting Werner - Partnerschaft von Patent- und Rechtsanwälten mbB

(57) **Abstract**

A vascular introducer sheath device comprising a tubular main shaft and a main hub attached to the proximal end of the main shaft. Attached to the proximal end of the main hub is a header hub, which has a first arm and a second arm. The header hub is attached to the main hub in a releasably locked configuration. As such, the header hub is functionally detachable from the main hub. The header hub and the main hub could together have a twist-on or snap-on mating mechanism. An example of such a mating mechanism is a male lock fitting on the header hub and a female lock fitting on the main hub. Further, there could be a side port on the main hub and a side port on the header hub, wherein the side ports for the main hub and header hub are oriented on a same plane when the header hub and the main hub are in locked configuration.

## Description

### Technical Field

This invention relates generally to vascular access introducer sheaths.

### Background

Various vascular access introducer sheaths have been introduced. However, the existing introducer sheaths do not allow the user to place a larger intravascular instrument during the procedure. Therefore, it is desirable that a vascular access introducer sheath be capable of having a releasable proximal hub for a direct access of the larger intravascular instrument without exchanging the introducer sheath during the procedure.

### Summary of Disclosure

In one aspect, the invention is a vascular introducer sheath device that comprises a tubular main shaft (11) having a shaft passage (14). Attached to the proximal end of the main shaft is a main hub (20) having a main hub passage (23). Attached to the proximal end of the main hub in a releasably locked configuration is a header hub (30) having a proximal end, a distal end, and a header passage (62).

The header hub is functionally detachable from the main hub. As used herein, "functionally detachable" means it has a mechanism specifically designed for intentional detachment by the operator in performing the medical procedure. The header hub and the main hub could be mated together by any suitable mating mechanism, such as a twist-on or snap-on mating mechanism.

The header hub may have two or more arms (31,41). The header hub could have a first arm and a second arm. The first arm has a first passage therein and the second arm has a second passage therein. The first passage (of the first arm) could be in coaxial orientation to the main shaft passage. The second passage (of the second arm) could be in angled orientation (e.g. 10-65°) to the header passage.

The various passages in the vascular introducer sheath device could be same or different diameters. For example, both the first passage (of the first arm) and second passage (of the second arm) could be narrower (internal diameter) than the header passage. In another example, the second passage (of the second arm) could be narrower (internal diameter) than the first passage (of the first arm).

In another aspect, the invention is a medical procedure kit that has components to assemble a vascular introducer sheath device. The kit comprises a first component that comprises a tubular main shaft and main hub as described herein. The kit further comprises a second component that is a header hub as described herein. The kit may further comprise other components such as a guidewire, needle, fluid tube with stopcock, etc.

In another aspect, the invention is a method of assembling a vascular introducer sheath device. The method comprises having a first component that comprises a tubular main shaft and main hub as described herein. The method further comprises having a second component that is a header hub as described herein. The method further comprises attaching the first component to the second component as described herein. The method may further comprise attaching a fluid tube with stopcock as described herein.

In another aspect, the invention is a method of establishing an intravascular access site in a patient. The method comprises having a vascular introducer sheath device comprising a tubular main shaft and a main hub attached to the proximal end of the main shaft. By percutaneous procedure, the introducer sheath device is inserted into a blood vessel of the patient such that the main shaft is inside the blood vessel, but the main hub is external so that it is accessible to the operator. This percutaneous procedure for inserting the introducer sheath device may be performed using a guidewire that is inserted into the blood vessel.

The method further comprises attaching a header hub to the proximal end of the main hub. The step of inserting by percutaneous procedure may be performed before or after the step of attaching the header hub to the main hub. For example, the vascular introducer sheath device may be fully assembled prior to the percutaneous insertion procedure or be partially assembled prior to the percutaneous insertion procedure.

The vascular introducer sheath device could be used in conjunction with intravascular instruments (51, 52, 53, 60). Examples of intravascular instruments include wires (e.g. guidewires), stents, balloons, catheters, etc. The intravascular instrument could be a type of catheter such as fluid infusion catheters, fluid aspiration catheters, mechanical actuation catheters (e.g. balloon catheters, intra-arterial "roto-rooter" cutting catheters), electrophysiology catheters (e.g. electrode catheters, radio-frequency ablation catheters), electronic sensor catheters (e.g. intravascular ultrasound catheters, optical coherence tomography catheters), etc.

The intravascular instruments being used in conjunction with the sheath device may be of different types from each other (e.g. different size, different function, different length, different material, etc.). That is, they are not necessarily identical copies of the same intravascular instrument. For example, one may be an electronic sensor catheter and the other may be a fluid infusion catheter.

In some embodiments, the method further comprises inserting a first intravascular instrument into the first arm; and advancing it through the header passage, through the main hub passage, and through the shaft passage. In some cases, the method further comprises inserting a second intravascular instrument into the second arm, and advancing it through the header passage, through the main hub passage, and through the shaft passage. The second intravascular instrument could be different from the first medical instrument. For example, the second intravascular instrument could be narrower than the first intravascular instrument.

The order of procedure steps for inserting intravascular instrument(s) into the introducer sheath device, attaching the main hub to the header hub, or detaching thereof may vary according to the particular situation. In some embodiments, the method further comprises, before attaching the header hub to the main hub, inserting a first intravascular instrument into the proximal end of the main hub, and advancing it through the main hub passage, and through the shaft passage. In some cases, the first intravascular instrument is withdrawn from the introducer sheath device, and after attaching the header hub to the main hub, a second intravascular instrument is inserted into the proximal end of the first or second arm, and advanced through the main hub passage, and through the shaft passage.

In some cases, the method further comprises withdrawing the first intravascular instrument from the introducer sheath device. The header hub is detached from the main hub so that a proximal opening of the main hub is exposed. A second intravascular instrument is inserted into the proximal opening of the main hub, and advanced through the main hub passage, and through the shaft passage. The second intravascular instrument could be different from the first intravascular instrument.

In some embodiments, the method further comprises inserting a first intravascular instrument into the first arm, and advancing it through a passage of the first arm, through the header passage, through the main hub passage, and through the shaft passage. While the first intravascular instrument is inside the sheath device, a second intravascular instrument is inserted into the second arm, and advanced through a passage of the second arm, through the header passage, through the main hub passage, and through the shaft passage.

In some cases, the method further comprises, before attaching the header hub to the main hub, inserting a third intravascular instrument into the proximal end of the main hub, and advancing it through the main hub passage, and through the shaft passage. The third intravascular instrument could be different from one or both the first and second intravascular instruments. In some cases, the method further comprises, before attaching the header hub to the main hub, withdrawing the third intravascular instrument from the introducer sheath device. After attaching the header hub to the main hub, the steps of inserting the first or second intravascular instruments into the introducer sheath device are performed.

Any use of the word "or" herein is intended to be inclusive and is equivalent to the expression "and/or," unless the context clearly dictates otherwise. As such, for example, the expression "A or B" means A, or B, or both A and B. Similarly, for example, the expression "A, B, or C" means A, or B, or C, or any combination thereof. The terms "first, second, etc." with respect to elements are being used herein only to distinguish one element from another element. But these are not intended to limit the elements in an ordinal fashion, such as defining the order, position, or priority of the elements. For example, a first element could be alternately be called a second element, and, similarly, a second element could alternately be called a first element, without departing from the scope of the present invention. The first element and the second elements are not the same element. The terms "first, second, etc." may refer to different elements in different embodiments.

### Brief Description of the Drawing

FIG. 1 shows a perspective view of an example introducer sheath according to the present invention;
FIG. 2 shows a side view of the introducer sheath;
FIG. 3 shows an internal view of the introducer sheath;
FIG. 4 shows a second hub as detached and isolated from the main hub of the introducer sheath;
FIG. 5 shows an internal view of the second hub;
FIG. 6 shows a proximal end of the second hub;
FIG. 7 shows a proximal end of the first hub;
FIG. 8A-8D show a locking mechanism;
FIG. 9 shows an internal view of the introducer sheath with two intravascular instruments inserted therein;
FIG. 10 shows an internal view of the short arm of the second hub with an intravascular instrument inserted into the introducer sheath;
FIG. 11 shows an internal view of the first hub with two intravascular instruments inserted into the introducer sheath; and
FIG. 12 shows an internal view of the introducer sheath without the second hub and with a larger intravascular instrument inserted therein.

### Detailed Disclosure

In the drawings, like numerals indicate like elements throughout. Certain terminology is used herein for convenience only and is not to be taken as a limitation on the present invention. The terms "distal" and "proximal" refer to the directions "away from" and "closer to," respectively, the body of the physician inserting the introducer sheath into a patient. The terminology includes the words above specifically mentioned, derivatives thereof, and words of similar import.

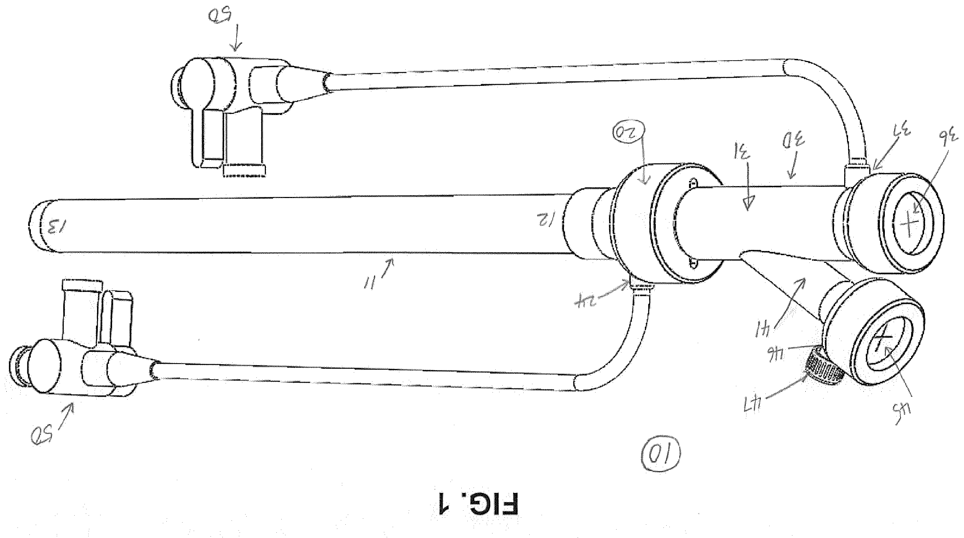

FIGS. 1, 2, and 4 show perspective and side views of an example embodiment of the introducer sheath according to the present invention. In this embodiment the introducer sheath 10 comprises a tubular member 11, a first hub 20, and a second hub 30. The first hub includes a side port 24 being connected to a three-way stopcock 50. The long arm 31 of the second hub 30 includes a side port 24 which is connected to another three-way stopcock 50. The short arm 41 of the second hub 30 has a flushing port 46 and its cap 47. Alternatively, the second hub may have more than two arms as necessary.

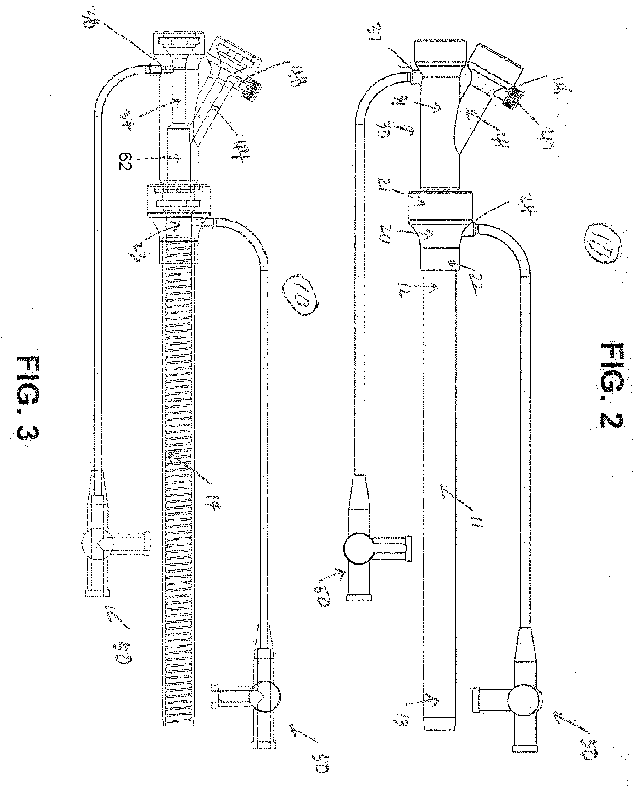

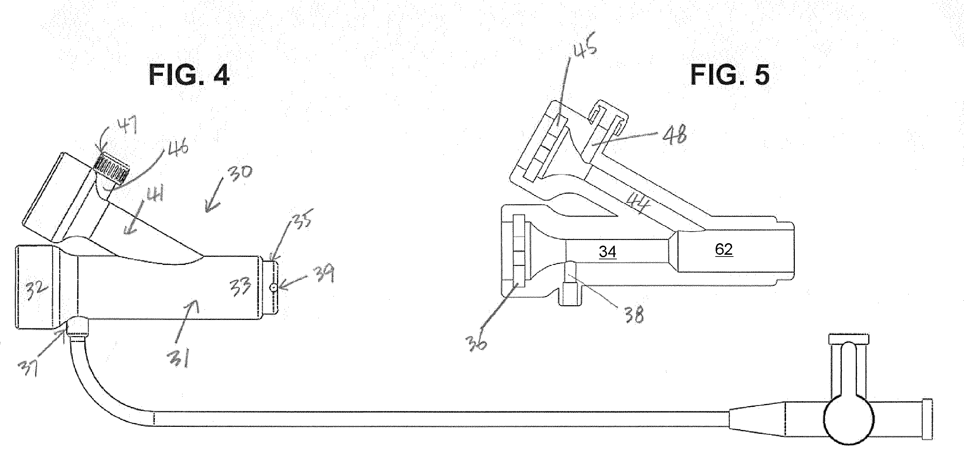

FIGS. 3 and 5 show an internal views of the introducer sheath 10. The short arm passage 44 merges into the long arm passage 34. The long arm passage 34 extends and conforms to the first hub passage 23 for communicating with the main passage 14 of the tubular member 11. The side port passage 25 is connected to the first hub passage 23, and the side port passage 38 is connected to the long arm passage 34. The second hub 30 has a passage 62 that is in communication with various other passages as shown. Also, the side port passage 48 is connected to the short arm passage 44.

FIG. 4 shows a second hub being detached from the introducer sheath. The distal end 33 of the second hub has a male lock fitting 35 for interlocking with the first hub 20. The male locking fitting 35 includes at least one protrusion 39 formed on the male lock fitting 35 to rotationally engage with the female lock fitting 27 of the first hub 20.

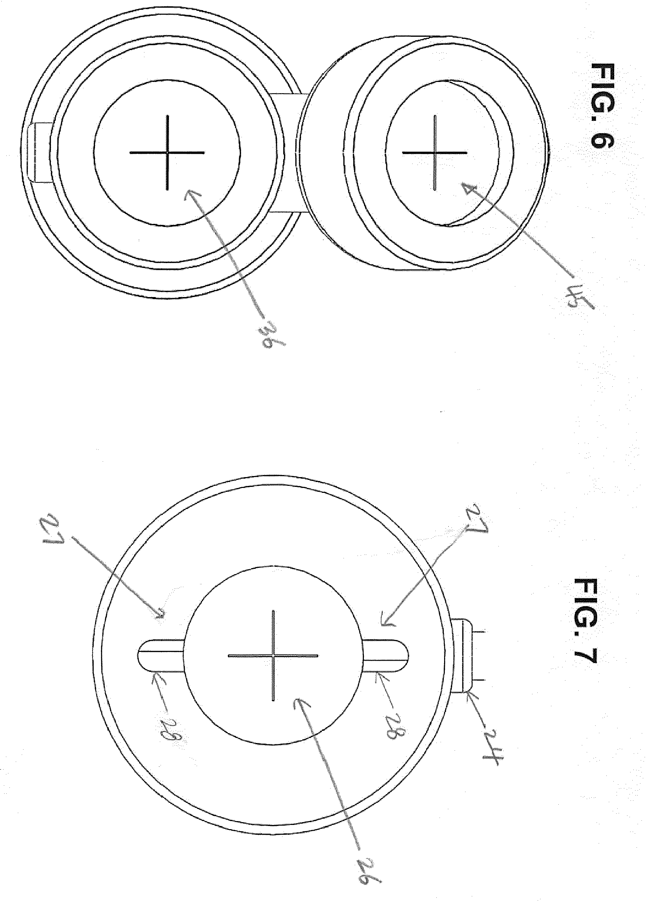

FIGS. 6 and 7 show proximal ends of the second hub and the first hub respectively. The hemostasis seal members 26, 36, 45 are disposed within the proximal ends of the first hub and second hub respectively to permit passage of an intravascular instrument therethrough, but are sufficiently self-sealing to substantially impede the outflow of blood.

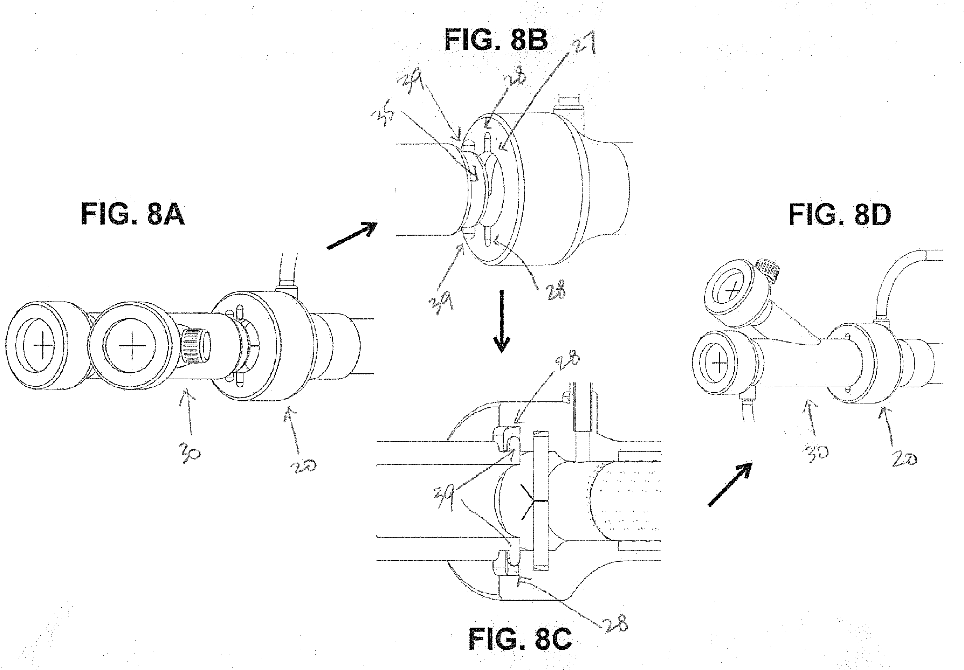

FIG. 7 also shows the female lock fitting 27 formed in the proximal end of the first hub 20. The female lock fitting 27 includes at least two lock grooves 28 to rotationally engage with the lock protrusion 29 of the second hub 30. For example, the lock protrusion 39 is pushed and then rotated about 90 degree for securely locking or completely releasing as illustrated in FIGS. 8A-8D. FIG. 8A illustrates a situation where the second hub 30 is unattached yet. FIG. 8B illustrates a situation where the second hub 30 is being inserted with the lock protrusion 39 aligned. FIG. 8C illustrates a situation where the second hub 30 is being rotated with 90 degree when inserted into the female lock fitting 27 of the first hub 20. FIG. 8D illustrates a situation where the second hub 30 is locked to the first hub 20.

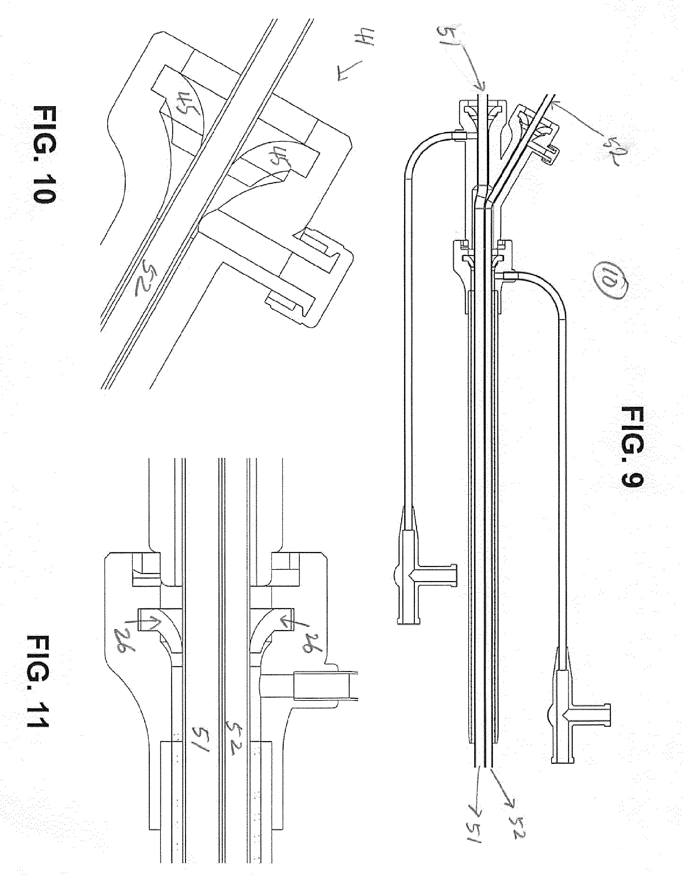

FIGS. 9-11 show an internal view of the introducer sheath when two intravascular instruments 51, 52 are placed. As shown in the FIGS. 9-11, each hemostasis seal member permits passage of the intravascular instrument while sealing, and the two instruments 51, 52 are passed through the main passage 14 of the tubular member 11.

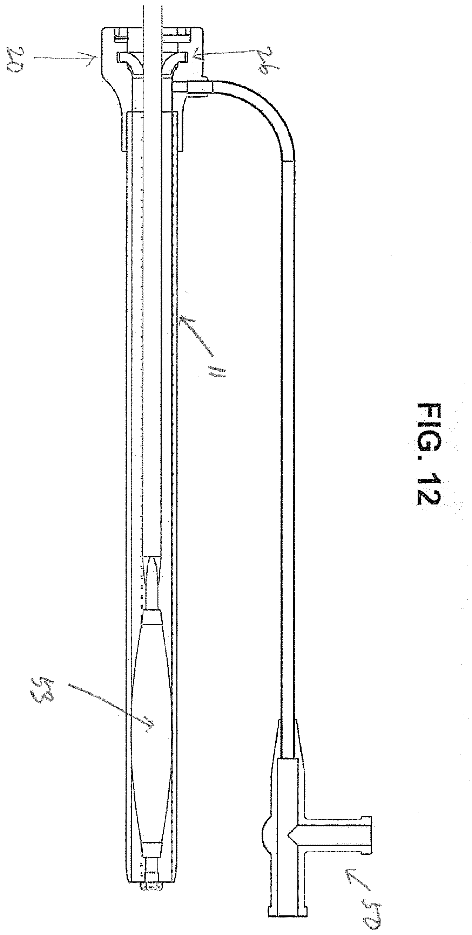

FIG. 12 shows an internal view of the introducer sheath without the second hub when a larger intravascular instrument 53 is placed. For example, when the intravascular instrument 53 is larger than the size of the long arm passage 34 and short arm passage 33, a surgeon can remove the second hub 30 from the introducer sheath 10 and place the intravascular instrument 53 directly into the first hub passage 23 which conforms to the main passage 14 as shown in FIG. 12.

### Tubular Member

As shown in FIGS 2 and 3, introducer sheath 10 comprises a tubular member 11 with proximal end 12, distal end 13, main passage 14, which extends longitudinally therethrough and opens at the distal and proximal ends for inserting intravascular instruments therein. Main passage 14 has a generally circular and relatively large cross-sectional area for accommodating a range of intravascular instruments. Tubular member 11 is formed of inelastic, semi-rigid plastic, or coiled materials. Preferably the tubular member 11 has a maximum of 30 French size.

### First Hub

As shown in FIGS. 9 and 11, introducer sheath 10 further comprises a first hub 20 with proximal end 21, distal end 22, first hub passage 23, which extends longitudinally therethrough. The first hub 20 is fixedly mounted on the proximal end 13 of the tubular member 11. Preferably, the first hub passage 23 conforms to the size and shape of the main passage 14 of the tubular member 11 for accommodating a range of intravascular instruments.

The first hub 20 includes a hemostasis seal member 26 disposed within the first hub 20 to permit passage of an intravascular instrument through the first hub passage 23. The first hub 20 also includes a side port 24 positioned between the hemostasis seal member 26 and distal end 22 of the first hub 20 for connecting a three-way stopcock 50. The side port 24 has a side port passage 25 which extends and communicates with the first hub passage 23 and the tubular member main passage 14. The first hub 20 further comprises a female lock fitting 27 formed in the proximal end 21 of the first hub 20.

### Long Arm of Second Hub

As shown in FIGS. 2-5, the introducer sheath 10 further comprises a second hub 30. The second hub 30 includes long arm 31 with proximal end 32, distal end 33, and long arm passage 34, which extends longitudinally therethrough and opens at the distal and proximal ends for inserting intravascular instruments therein. Long arm passage 34 has a longitudinal axis that is at least substantially parallel to the longitudinal axis of first hub passage 23 and main passage 14 of the tubular member 11. In this way, main passage 14 communicates with long arm passage 34 for in-line introduction of intravascular instruments or fluid therethrough.

Long arm passage 34 has a generally circular and relatively large cross-sectional area for accommodating a range of intravascular instruments. Long arm 31 is formed of inelastic, semi-rigid plastic, or coiled materials. Preferably, long arm passage 34 has about 12 French size at the proximal end 32 and gradually expands to about 22 French size at the distal end of 33 to conform to the size and shape of the first hub passage 23 and the tubular member main passage 14.

Long arm 31 includes a hemostasis seal member 36 disposed within the long arm 31 to permit passage of an intravascular instrument through the long arm passage 34. Long arm 31 also includes a side port 37 positioned between the hemostasis seal member 36 and distal end 33 of the long arm 31. The side port 37 has a side port passage 38, which extends and further communicates with the long arm passage 34 and the tubular member main passage 14. As seen here, side port 37 and side port 24 are oriented on the sheath device such that they are on the same plane when the sheath device is in locked configuration.

Long arm 31 also includes a male lock fitting 35 for lockable attachment to the female lock fitting 27 of the first hub 20. The second hub 30 are releasably attached and secured with the first hub 20. In contrast, the second hub 30 are unlocked and completely detached from the first hub 20 by turning 90 degree. Accordingly, when a larger intravascular instrument 60 is unable to pass through either first or short arm of the second hub 30, the larger intravascular instrument 60 can directly be inserted into the first hub 20 without exchanging the introducer sheath during the procedure by unlocking and removing the second hub 30 from the first hub 20.

### Short Arm of Second Hub

As shown in FIGS. 9 and 10, the second hub 30 includes a short arm passage 44 positioned in a short arm 41 for communicating with long arm passage 34 and extending laterally from the longitudinal axis of the main passage 14 of the tubular member 11. Short arm passage 44 provides for the introduction of an intravascular instrument or fluid into the tubular member main passage 14 when another intravascular instrument extends from the main passage 14 through the first hub passage 34 of the second hub 30.

Short arm 41 further includes a hemostasis seal member 45 disposed within the short arm 41 to permit passage of an intravascular instruments through the short arm passage 44. Short arm 41 further includes a flushing port 46 and cap 47 positioned between the hemostasis seal member 45 and distal end 43 of the short arm 41 for connecting a three-way stopcock 50. The flushing port 46 has a flushing passage 48, which extends and communicates with the long arm passage 34 of the second hub 30.

## Claims

1. A vascular introducer sheath device comprising:
a tubular main shaft having a proximal end, a distal end, and a shaft passage;
a main hub having a proximal end, a distal end, and a main hub passage; wherein the main hub is attached to the proximal end of the main shaft;
a header hub having a proximal end, a distal end, and a header passage; wherein the header hub has a first arm and a second arm;
wherein the header hub is attached to the proximal end of the main hub in a releasably locked configuration, and wherein the header hub is functionally detachable from the main hub.

2. The device of claim 1, wherein the header hub and the main hub together have a twist-on or snap-on mating mechanism.

3. The device of claim 2, wherein the mating mechanism is a male lock fitting at the distal end of the header hub and a female lock fitting at the proximal end of the main hub that is designed to receive the male lock fitting of the header hub.

4. The device of claim 3, wherein the distal end of the header hub further comprises a lock protrusion, and the proximal end of the main hub comprises a slot and groove; wherein the lock protrusion inserts through the slot and is slidable within the groove.

5. The device of claim 1, wherein the first arm has a first passage that is in coaxial orientation to the shaft passage.

6. The device of claim 5, wherein the second arm has a second passage that is in angled orientation to the header passage.

7. The device of claim 6, wherein both the first passage and second passage are narrower than the header passage.

8. The device of claim 7, wherein the second passage is narrower than the first passage.

9. The device of claim 6, wherein the second arm has a proximal opening, a flush port, and a removable cap on the flush port.

10. The device of claim 1, further comprising a side port on the main hub and a side port on the header hub, wherein the side ports for the main hub and header hub are oriented on a same plane when the header hub and the main hub are in locked configuration.

11. The device of claim 1, wherein each of the proximal end of the main hub, proximal end of the first arm, and proximal end of the second arm has an opening with a hemostatic seal.
